# FASCICULE DE BREVET EUROPEEN

(11) **EP 3 077 061 B1**
(45) Date de publication et mention de la délivrance du brevet: **18.03.2020**
(21) Numéro de dépôt: 14806269.8
(22) Date de dépôt: 03.12.2014
(51) Int. Cl.: A61Q 19/08, A61K 31/00, A61K 31/728, A61K 36/28, A61P 17/04, A61P 17/10

(54) **COMPOSITION POUR REDUIRE LES TROUBLES DE LA SENESCENCE CUTANEE COMPRENANT UN RÉTINALALDÉHYDE ET UN EXTRAIT DE LEONTOPODIUM ALPINUM**
ZUSAMMENSETZUNG ZUR REDUKTION DER HAUTALTERUNG UMFASSEND RETINALALDEHYD UND EINEN EXTRAKT VON LEONTOPODIUM ALPINUM
COMPOSITION FOR REDUCING SKIN AGING COMPRISING RETINALALDEHYDE AND AN EXTRACT OF LEONTOPODIUM ALPINUM

(30) Priorité: 03.12.2013 FR 1362015
(43) Date de publication de la demande: 12.10.2016
(73) Titulaire: Galephar M/F, 6900 Marche-en-Famenne (BE)
(72) Inventeur: BAUDIER, Philippe, B-1180 Bruxelles (BE)
(74) Mandataire: Office Kirkpatrick
(86) Numéro de dépôt international: PCT/EP2014/076356
(87) Numéro de publication internationale: WO 2015/082520

(56) Documents cités:
- EP-A1- 2 522 330
- WO-A1-01/87256
- FR-A1- 2 782 919
- FR-A1- 2 980 361
- JP-A- 2002 138 027
- US-A1- 2012 035 130
- Anonymous: "REVELATION", , 4 janvier 2013 (2013-01-04), pages 1-2, XP055115517, Extrait de l'Internet: URL:http://web.archive.org/web/20130104084 338/http://www.sircuitskin.com/inc/sdetail /32506/43667 [extrait le 2014-04-28]
- DATABASE GNPD [Online] MINTEL; janvier 2013 (2013-01), anonymous: "Night Serum With Retinol", XP002723753, extrait de www.gnpd.com accession no. 1947053 Database accession no. 1947053
- Anonymous: "SIRCTUIT cosmeceuticals - Ingredient Glossary", , 4 février 2013 (2013-02-04), pages 1-8, XP055115539, Extrait de l'Internet: URL:http://web.archive.org/web/20130204081 955/http://www.sircuitskin.com/ingredient_ glossary [extrait le 2014-04-28]
- Asia Anonymous ET AL: "ALPAFLOR EDELWEISS", , 1 janvier 2013 (2013-01-01), pages 1-2, XP055115585, Extrait de l'Internet: URL:http://www.innovadex.com/documents/118 8142.pdf?bs=472&b=129346&st=1&sl=28571595& crit=a2V5d29yZDpbYWxwYWZsb3IgZWRlbHdlaXNzX Q==&k=alpaflor|edelweiss [extrait le 2014-04-28]
- LULLI DANIELA ET AL: "Anti-Inflammatory Effects of Concentrated Ethanol Extracts of Edelweiss (Leontopodium alpinum Cass.) Callus Cultures towards Human Keratinocytes and Endothelial Cells", MEDIATORS OF INFLAMMATION, vol. 2, no. 4, 1 janvier 2012 (2012-01-01) , pages 183-12, XP055115622, ISSN: 0962-9351, DOI: 10.1089/ars.2005.7.889
- DOBNER M J ET AL: "Anti-inflammatory activity of Leontopodium alpinum and its constituents", PLANTA MEDICA, THIEME VERLAG, DE, vol. 70, no. 6, 1 juin 2004 (2004-06-01), pages 502-508, XP002549565, ISSN: 0032-0943, DOI: 10.1055/S-2004-827148
- CHOU T C ET AL: "Quantitative analysis of dose-effect relationships: the combined effects of multiple drugs or enzyme inhibitors", ADVANCES IN ENZYME REGULATION, PERGAMON PRESS, OXFORD, GB, vol. 22, 1 janvier 1984 (1984-01-01), pages 27-55, XP023796270, ISSN: 0065-2571, DOI: 10.1016/0065-2571(84)90007-4 [extrait le 1984-01-01]
- DATABASE GNPD [Online] MINTEL; novembre 2011 (2011-11), Anonymous: "Eye Cream - Glo Therapeutics", XP002735483, extrait de www.gnpd.com Database accession no. 1676605

## Description

### Domaine technique

La présente invention concerne une composition comprenant au moins un rétinaldéhyde et/ou un de ses stéréostéréoisomères et au moins un extrait de *Leontopodium alpinum.* La composition de l'invention peut-être notamment destinée à un usage cosmétique, dermatologique ou pharmaceutique, plus particulièrement pour la prévention ou le traitement des troubles de la sénescence cutanée associés au vieillissement physiologique de la peau et/ou au vieillissement actinique de la peau et en particulier pour réduire la production d'agents oxydants et augmenter la synthèse de collagène.

### Arrière-plan technologique

La peau humaine est constituée de deux tissus l'un superficiel, l'épiderme, l'autre profond, le derme. L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau, notamment le rôle de protection de l'organisme des agressions extérieures (climat, rayons ultraviolets, tabac...), appelé " fonction barrière ". Le derme fournit à l'épiderme un support solide. C'est également son élément nourricier. Il est principalement constitué de fibroblastes et d'une matrice extracellulaire composée majoritairement de collagène, d'élastine et d'une substance, dite substance fondamentale. Ces composants sont synthétisés par les fibroblastes. On y trouve aussi des leucocytes, des mastocytes ou encore des macrophages tissulaires. Enfin, le derme est traversé par des vaisseaux sanguins et des fibres nerveuses. La cohésion entre l'épiderme et le derme est assurée par la jonction épidermique. C'est une région complexe d'une épaisseur de 100 nm environ qui comprend le pôle basal des kératinocytes basaux, la membrane épidermique et la zone sous basale du derme superficiel (Bernard P. Structure de la jonction dermo-épidermique. Objectif peau. 2001, 68 : 87-93). D'un point de vue structurel, des hémidesmosomes, dans lesquels s'insèrent des filaments de kératine (complexe hémidesmosome-tonofilaments), sont répartis sur la membrane plasmique des kératinocytes basaux. Au regard de ces complexes hémidesmosome-tonofilaments, on trouve des filaments d'ancrage qui traversent la membrane basale épidermique. Les filaments d'ancrage sont reliés à la laminine 5 côté épidermique. Enfin, des fibrilles d'ancrage constituent le réseau sous basal. Ce sont des structures curvilinéaires qui prennent naissance et se terminent sur la face profonde de la membrane basale et dans lesquelles viennent s'engager des fibres de collagène I, III et V. Il a été montré que ces fibrilles d'ancrage, parfaitement visualisables en microscopie électronique, sont composées de collagène de type VII (ci-après collagène VII). Le collagène VII est synthétisé par les kératinocytes et les fibroblastes mais de façon plus importante par les kératinocytes (Aumailley M, Rousselle P. laminins of the dermoepidermal junction. Matrix Biology. 1999, 18 : 19-28 ; Nievers M, Schaapveld R, Sonnenberg A. Biology and function of hemidesmosomes, Matrix Biology, 1999, 18 : 5- 17).

Ainsi, les collagènes sont les protéines majoritaires des matrices extracellulaires. A ce jour, 20 types de collagènes sont identifiés et notés de I à XX. On distingue différentes familles de collagène parmi ces types en fonction des structures formées :
- la famille des collagènes fibrillaires (type I, II, II V/XI) qui forment donc des fibres ;
- la famille des collagènes formant le réseau des 5 membranes basales qui comprend le collagène de type VI et de type XVII ;
- les collagènes formant des réseaux hexagonaux (type VIII et type X), des filaments perlés (Type VI), des FACIT (type IX, XII, XIV, XVI, XIX, XX) les fibrilles d'ancrage qui correspondent au type VII;
- les multiplexines (type XV, XVII) et le collagène de type XIII dont on ne connaît pas les fonctions précises à ce jour.

Dans la peau, les collagènes majoritairement présents dans l'ensemble du derme sont les collagènes de type I et III qui forment la matrice extracellulaire de l'ensemble du derme (ce sont ces collagènes qui constituent 70-80 % du poids sec du derme).

Par ailleurs, les collagènes ne sont pas tous synthétisés par les mêmes types cellulaires, les collagènes de type I et III sont essentiellement produits par le fibroblaste dermique alors que le collagène de type VII est produit par le kératinocyte épidermique. Enfin, la régulation de leur expression diffère d'un collagène à un autre, par exemple, les collagènes I et VII ne sont pas régulés de la même manière par certaines cytokines, en effet, le TNF alpha et la leukoreguline stimulent le collagène VII et régulent négativement le collagène I.

Enfin, toutes les molécules de collagène sont des variantes d'un précurseur commun qui est la chaîne alpha du procollagène.

Avec le vieillissement, le collagène s'amincit et des rides apparaissent à la surface de la peau. Le vieillissement cutané est un mécanisme génétiquement programmé.

Par ailleurs, certains facteurs d'environnement, comme l'exposition au rayonnement du soleil, accélèrent le processus de vieillissement Une exposition au soleil répétée entraine la formation de radicaux libres. Dans l'organisme, cette réaction est appelée stress oxydatif. La production d'espèces d'oxygène réactives provoque donc des dommages au niveau de l'ADN, des protéines ou des lipides, contribuant notamment à accélérer le vieillissement cellulaire de la peau et/ou des phanères. Sous l'effet du rayonnement UV, des dommages à l'ADN sont induits favorisant la formation de cellules photo-endommagées de la peau qui constituent un des signes du vieillissement cutané.

En particulier, les effets du stress oxydatif affectent la respiration cellulaire et se traduisent notamment par un vieillissement accéléré de la peau, avec en particulier un teint terne et/ou gris, un teint inhomogène, une perte de radiance et/ou de transparence de la peau, la formation précoce de rides ou ridules, notamment une perte de douceur, de souplesse et d'élasticité de la peau, l'apparition de tâches pigmentaires, en particulier de lentigo actiniques.

La peau a ainsi un aspect beaucoup plus âgé sur les zones exposées au soleil comme le dos des mains ou le visage.

Il existe par conséquent un besoin croissant pour des compositions visant à pallier les signes du vieillissement cutané.

Le document SIRCUIT cosmeceuticals Anonymous « REVELATION » (2013-01-04), p.1-2,XP055115517 (web.archive.org/web/20130104084338/ http://www .sircuitskin.com/inc/sdeta décrit un sérum anti rides contour des yeux, renfermant en particulier une culture de cellules de méristème de Leontopodium Alpinum et de la vitamine A (rétinol), ainsi que 8 autres principes actifs (peptides, beta glucane, arginine, tocotriénols, antarcticine et une source d'acide betulinique).

La demande US 2012/035130 A1 décrit des compositions destinées à clarifier la peau et aussi à traiter divers autres symptômes disgracieux dont les rides, renfermant en particulier un mélange de gamma-cyclodextrine et de rétinal, ainsi que le cas échéant d'autres ingrédents, par exemple des extraits de plantes.

### Résumé de l'invention

La présente invention a précisément pour but de proposer une nouvelle composition synergique susceptible d'être utilisée dans le domaine cosmétique ou pharmaceutique pour limiter le vieillissement de la peau, qu'il soit physiologique ou actinique, et notamment le vieillissement généré par la production de radicaux libres, une diminution de l'élasticité de la peau et/ou par une dégradation du collagène dans la structure des tissus.

De façon surprenante, la Demanderesse a constaté qu'une composition comprenant au moins un rétinaldéhyde et/ou un de ses stéréoisomères et au moins un extrait de *leontopodium alpinum* était capable d'augmenter la synthèse de collagène et de diminuer la production de radicaux libres, permettant ainsi de contrer les signes du vieillissement.

La présente invention concerne donc une composition, comprenant au moins un rétinaldéhyde et/ou un de ses stéréoisomères et au moins un extrait de *leontopodium alpinum* , lequel extrait, selon l'invention, est constitué de 0,01 à 2% en poids d'une préparation de cellules d'une culture de cellules de méristème de *leontopodium alpinum* en présence de glycérine et de gomme xanthane, disponible sous la marque Leontopod stems GX®, contenant 20% de cellules, 0.3% de gomme xanthane et pour le reste de la glycérine, par rapport au poids total de la composition.

Dans un mode de réalisation particulier, le rétinaldéhyde de l'invention est sélectionné parmi le groupe comprenant : le rétinaldéhyde trans-cis, le rétinaldéhyde tout-trans ou un mélange de ses deux stéréoisomères.

Dans un mode de réalisation particulier, le rétinaldéhyde représente de 0,01 à 2% en poids, de préférence de 0,05 à 0,1% en poids par rapport au poids total de la composition.

Dans un mode de réalisation particulier, l'extrait de *leontopodium alpinum* selon l'invention représente de préférence de 0,05 à 2% en poids par rapport au poids total de la composition.

Dans un mode de réalisation particulier, la composition comprend en outre au moins un autre principe actif sélectionné parmi le groupe consistant en : le crotamiton, l'acide azélaïque, l'arbutine et le hyaluronate de sodium.

Dans un mode de réalisation particulier, le hyaluronate de sodium est un hyaluronate de sodium de haut poids moléculaire supérieur à 750000 daltons.

Dans un mode de réalisation particulier, le principe actif supplémentaire représente de 0,1 à 2% en poids, de préférence de 0,5 à 1% en poids par rapport au poids total de la composition.

D'autres caractéristiques et avantages de la présente invention apparaîtront à la lecture de la description détaillée qui suit et du mode de réalisation préféré de l'invention, donnée à titre d'exemple.

### Description détaillée

On entend par « cosmétiquement ou pharmaceutiquement acceptable », des moyens adaptés à une utilisation en contact avec la peau et en particulier les cellules de l'épiderme, sans toxicité, incompatibilité, instabilité, irritation, réponse allergique ou similaire, avec un rapport bénéfice / risque raisonnable.

On entend par « troubles de la sénescence cutanée », « signes du vieillissement de la peau » ou « signes cutanés du vieillissement », toutes modifications de l'aspect extérieur de la peau dues au vieillissement qu'il soit physiologique et/ou actinique, comme par exemple les rides et ridules, la peau flétrie, la peau molle, la peau amincie, la peau terne et sans éclat, le manque d'élasticité et/ou de tonus de la peau, mais également toutes modifications internes de la peau qui ne se traduisent pas systématiquement par un aspect extérieur modifié, comme par exemple toutes dégradations internes de la peau, particulièrement des fibres de collagène, consécutives à une exposition aux rayonnements ultra-violets, qui peuvent avoir comme conséquence l'amincissement du derme.

La Demanderesse a trouvé, de façon surprenante et inattendue, que la combinaison synergique d'un rétinaldéhyde avec un extrait de *leontopodium alpinum* permet de réduire l'apparition des signes du vieillissement cutanée et notamment l'apparition des rides, en stimulant la régénération du collagène et réduisant la peroxydation lipidique induite par les radicaux libres.

De manière préférée, le rétinaldéhyde ou « rétinal » selon l'invention est un dérivé naturel de la vitamine A. De manière préférée, le rétinaldéhyde selon l'invention se présente sous la forme 13 trans (tout-trans) ou 13 cis (trans- cis) ou sous la forme d'un mélange de ses deux stéréoisomères.

Le rétinaldéhyde présent dans la composition selon l'invention représente de 0,01 à 2% en poids par rapport au poids total de la composition. De manière préférée, le rétinaldéhyde représente de 0,05 à 0,1% en poids par rapport au poids total de la composition. De manière préférée, le rétinaldéhyde présent dans la composition selon l'invention représente de 0.02 à 0,2% en poids, de préférence de 0,05 à 0,1% en poids de rétinaldéhyde par rapport au poids total de la composition.

La composition selon l'invention comprend également une préparation de cellules d'une culture de cellules de méristème de *Leontopodium alpinum.* Le *Leontopodium alpinum* ou Edelweiss est notamment connu pour ces propriétés anti-inflammatoires et anti-oxydantes. Des recherches récentes ont démontré que le pouvoir antioxydant du *leontopodium alpinum* était nettement supérieur à celui des produits classiquement utilisés, tels que la vitamine E ou le rétinol.

Selon l'invention, l'extrait de *leontopodium alpinum* utilisé dans la composition de l'invention est une préparation de cellules en culture in vitro de cellules dédifférenciées de leontopodium alpinum, à savoir d'une culture de cellules de méristème de *leontopodium alpinum.* La préparation de cellules dédifférenciées de *Leontopodium alpinum* est décrite dans l'art antérieur et en particulier dans le texte de la demande WO2001047538.

Des feuilles de *leontopodium alpinum* sont prélevées et découpées puis placées en boite de Pétri sur milieu nutritif. Des cals primaires apparaissent entre 2 et 5 semaines. Au fur et à mesure des repiquages, les cellules se stabilisent et sont transférées dans le même milieu de culture liquide en fermenteur. Les cellules obtenues en milieu liquide sont récupérées par filtration puis sont placées à -20°C en congélation lente propice à la formation de cristaux intracellulaires de grande taille permettant de casser les cellules.

L'extrait de *leontopodium alpinum* présent dans la composition selon l'invention correspond à une préparation de cellules d'une culture de cellules de méristème de *leontopodium alpinum* en présence de glycérine et de gomme xanthane, par exemple disponible sous la marque Leontopod stems GX® contenant 20% de cellules, 0.3% de gomme xanthane et pour le reste de la glycérine.

L'extrait de *leontopodium alpinum* présent dans la composition selon l'invention représente de 0,01 à 2% en poids, de préférence de 0,05 à 2% en poids d'extrait de *leontopodium alpinum* par rapport au poids total de la composition. De manière préférée, l'extrait de *leontopodium alpinum* présent dans la composition selon l'invention représente de 0,2 à 2% en poids, de préférence de 0,5 à 1% en poids d'extrait de *leontopodium alpinum* par rapport au poids total de la composition.

Selon un mode de réalisation particulier, la composition selon l'invention comprend en outre un véhicule cosmétiquement ou pharmaceutiquement acceptable.

Par « véhicule cosmétiquement ou pharmaceutiquement acceptable », on entend toute substance adaptée à une utilisation en contact avec la peau, et en particulier les cellules de l'épiderme, sans toxicité, incompatibilité, instabilité, irritation, réponse allergique ou similaire, avec un rapport bénéfice / risque raisonnable.

La composition selon l'invention peut se présenter sous toutes les formes galéniques classiquement utilisées, et notamment sous forme d'une solution ou suspension aqueuse, alcoolique ou hydroalcoolique, ou huileuse; d'une solution ou d'une dispersion du type lotion ou sérum; d'une émulsion, notamment de consistance liquide ou semi-liquide, du type HIE, E/H ou multiple; d'une suspension ou émulsion de consistance molle de type crème (H/E) ou (E/H); d'un gel aqueux ou anhydre, ou de toute autre forme cosmétique ou pharmaceutique.

La forme de la composition cosmétique ou pharmaceutique selon l'invention n'est pas particulièrement limitée, et la composition cosmétique ou pharmaceutique selon l'invention peut être dans une forme quelconque, telle qu'un liquide, une pâte, un gel, un solide, ou une poudre. La composition cosmétique ou pharmaceutique de l'invention est spécifiquement de préférence formulée en une composition cosmétique ou pharmaceutique pour la peau et des exemples de celle-ci comprennent des lotions pour la peau, des lotions, des crèmes, des émulsions, des sérums, des vernis, des fonds de teint, des eyeliners, des crayons pour sourcils, des mascaras, des ombres à paupières, des rouges à lèvres, des sticks à lèvres, des poudres pour le visage, des poudres et des agents d'écran solaire.

La composition selon l'invention peut être aqueuse ou anhydre.

Lorsque la composition se présente sous forme aqueuse, notamment sous forme de dispersion, d'émulsion ou de solution aqueuse, elle peut comprendre une phase aqueuse, qui peut comprendre de l'eau, une eau florale et/ou une eau minérale. La composition est de préférence une composition aqueuse et comprend alors de l'eau à une concentration de préférence comprise entre 5 et 98% en poids, notamment 20 et 95% en poids, mieux 50 et 95% en poids, par rapport au poids total de la composition.

Ladite phase aqueuse peut comprendre en outre un ou plusieurs solvants organiques tels qu'un alcool en notamment l'éthanol, l'isopropanol, le tert-butanol, le n-butanol, des polyols comme la glycérine, le propylène glycol, le butylène glycol, l'isoprène glycol, le polyéthylène glycol, macrogol et des éthers de polyol.

Lorsque la composition selon l'invention se présente sous la forme d'une émulsion, elle peut éventuellement comprendre en outre un tensioactif, de préférence en une quantité de 0,01 à 30 % en poids par rapport au poids total de la composition. La composition selon l'invention peut également comprendre au moins un co-émulsionnant qui peut être choisi parmi le monostéarate de sorbitan éthoxylé, le monostérate de sorbitan, le monostérate de glycérol, des alcools gras tels que l'alcool stéarylique ou l'alcool cétylique, ou des esters d'acides gras et de polyols tels que le stéarate de glycéryle.

La composition selon l'invention peut également comprendre une phase grasse, notamment constituée de corps gras liquides à 25°C, tels que des huiles d'origine animale, végétale, minérale ou synthétique, volatiles ou non; de corps gras solides à 25°C tels que des cires d'origine animale, végétale, minérale ou synthétique; de corps gras pâteux; de gommes; de leurs mélanges.

Les huiles volatiles sont généralement des huiles ayant, à 25°C, une tension de vapeur saturante au moins égale à 0,5 millibar (soit 50 Pa).

Parmi les constituants de la phase grasse, on peut citer :
- les silicones volatiles cycliques ayant de 3 à 8 atomes de silicium, de préférence de 4 à 6.
- les cyclocopolymères du type diméthylsiloxane/méthylalkylsiloxane,
- les silicones volatiles linéaires ayant de 2 à 9 atomes de silicium.
- les huiles volatiles hydrocarbonées, telles que les isoparaffines et notamment l'isododécane et des huiles fluorées.
- les polyalkyl(C1-C20) siloxanes et notamment ceux à groupements terminaux triméthylsilyle, parmi lesquels on peut citer les polydiméthylsiloxanes linéaires et les alkylméthylpolysiloxanes tels que la cétyldiméthicone (nom CTFA),
- les silicones modifiées par des groupements aliphatiques et/ou aromatiques, éventuellement fluorés, ou par des groupements fonctionnels tels que des groupements hydroxyles, thiols et/ou amines.
- les huiles de silicone phénylées,
- les huiles d'origine animale, végétale ou minérale, et notamment les huiles animales ou végétales formées par des esters d'acide gras et de polyols, en particulier les triglycérides liquides, par exemple les huiles de tournesol, de maïs, de soja, de courge, de pépins de raisin, de sésame, de noisette, d'abricot, d'amandes, de carthame ou d'avocat; les huiles de poisson (squalane), le tricaprocaprylate de glycérol, ou les huiles végétales ou animales de formule dans laquelle représente le reste d'un acide gras supérieur comportant de 7 à 19 atomes de carbone et R2 représente une chaîne hydrocarbonée ramifiée contenant de 3 à atomes de carbone, par exemple, l'huile de Purcellin ; l'huile de paraffine, de vaseline, le perhydrosqualène, l'huile de germes de blé, de calophyllum, de sésame, de macadamia, de pépins de raisin, de colza, de coprah, d'arachide, de palme, de ricin, de jojoba, d'olive ou de germes de céréales ; des esters d'acides gras ; des alcools ; des acétylglycérides ; des 10 octanoates, décanoates ou ricinoléates d'alcools ou de polyalcools ; des triglycérides d'acides gras (comme par exemple Neobee M5); des glycérides ;
- les huiles fluorées et perfluorées ;
- les gommes de silicones ;
- les cires d'origine animale, végétale, minérale ou synthétique telles que les cires microcristallines, la paraffine, le pétrolatum, la vaseline, l'ozokérite, la cire de montan; la cire d'abeilles, la lanoline et ses dérivés; les cires de Candellila, d'Ouricury, de Carnauba, du Japon, le beurre de cacao, les cires de fibres de lièges ou de canne à sucre ; les huiles hydrogénées concrètes à 25°C, les ozokérites, les esters gras et les glycérides concrets à 25 °C ; les cires de polyéthylène et les cires obtenues par synthèse de Fischer-Tropsch ; des huiles hydrogénées concrètes à 25 °C ; des lanolines ; des esters gras concrets à 25°C ; les cires de silicone ; les cires fluorées.

La composition selon l'invention peut en outre comprendre un autre principe actif. Ce principe actif supplémentaire est utilisé pour traiter et/ou renforcer l'action du rétinaldéhyde et/ou un de ses stéréoisomères et de l'extrait de *Leontopodium alpinum* dans une affection telle que l'acné.

Le principe actif supplémentaire est de préférence choisi parmi le groupe consistant en : le crotamiton, l'acide azélaïque, l'arbutine et le hyaluronate de sodium.

Ainsi, l'ajout de crotamiton dans la composition de l'invention permet notamment de traiter les prurits d'étiologies variées et la dermatite atopique.

L'ajout de l'acide azélaïque dans la composition de l'invention permet notamment de traiter l'acné par voie topique, en inhibant la thiorédoxine réductase (enzyme membranaire), dont le substrat est un inhibiteur de la tyrosinase.

L'arbutine a une action dans le traitement des zones hyperpigmentées et les taches brunes, associée à une exposition prolongée au rayonnement du soleil. En effet, elle a pour effet de diminuer le taux de mélanine et de blanchir la peau et les marques liées aux coups de soleil. L'ajout d'un tel principe actif dans la composition de l'invention permet de renforcer l'action de la composition dans le traitement des signes du vieillissement.

Le hyaluronate de sodium de haut poids moléculaire (supérieur à 750 000 daltons) est un agent hydratant, notamment utilisé pour favoriser l'hydratation, et pour stimuler la cicatrisation et les défenses naturelles de la peau. De par son action hydratante sur la peau, l'ajout d'un tel principe actif dans la composition de l'invention permet de renforcer l'action de la composition dans le traitement des signes du vieillissement, et notamment son action anti-rides.

Le principe actif supplémentaire présent dans la composition selon l'invention représente de 0,1 à 2% en poids, de préférence de 0,5 à 2% en poids par rapport au poids total de la composition. De manière préférée, le principe actif supplémentaire présent dans la composition selon l'invention représente de 0,2 à 2% en poids, de préférence de 0,5 à 1% en poids par rapport au poids total de la composition.

De façon connue, la composition selon l'invention peut comprendre les adjuvants habituels dans le domaine considéré, tels que les gélifiants hydrophiles ou lipophiles, les additifs hydrophiles ou lipophiles, les actifs notamment cosmétiques ou pharmaceutiques hydrophiles ou lipophiles, les conservateurs, les épaississants, les vitamines, les régulateurs de pH, les antioxydants, les solvants, les parfums, les charges, les pigments, les nacres, les filtres UV, les absorbeurs d'odeur et les colorants. Ces adjuvants, selon leur nature, peuvent être introduits dans la phase grasse, dans la phase aqueuse et/ou dans des sphérules lipidiques.

La nature et la quantité de ces adjuvants peuvent être choisies par l'homme du métier, sur la base de ses connaissances générales, de manière à obtenir la forme de présentation désirée pour la composition. En tout état de cause, l'homme du métier veillera à choisir tous les éventuels composés complémentaires et/ou leur quantité, de manière telle que les propriétés avantageuses de la composition selon l'invention ne soient pas, ou substantiellement pas, altérées par l'adjonction envisagée.

La composition selon l'invention peut être utilisée pour réduire les troubles de la sénescence cutanée.

La composition selon l'invention peut également être utilisée pour traiter une affection telle que l'acné.

La composition selon l'invention peut être utilisée dans une méthode de traitement cosmétique non-thérapeutique de la peau, consistant en l'application sur la peau de ladite composition, à laisser celle-ci en contact puis éventuellement à rincer.

Cette méthode de traitement cosmétique non-thérapeutique peut être mise en œuvre notamment en appliquant la composition cosmétique telle que définie ci-dessus, selon la technique d'utilisation habituelle de la composition, par exemple : application de crèmes, de gels, de sérums, de lotions, de laits, de démaquillage ou de compositions antisolaires.

Les exemples et modes de réalisation décrits et représentés illustrent l'invention, laquelle est susceptible de nombreuses variantes accessibles à l'homme de l'art.

### Exemples :

Les exemples et compositions suivants illustrent l'invention sans la limiter aucunement. Dans les compositions les proportions indiquées sont des pourcentages en poids. Les extraits de *Leontopodium alpinum* utilisés dans les exemples correspondent une préparation de cellules d'une culture de cellules de méristème de *leontopodium alpinum* en présence de glycérine et de gomme xanthane, disponible sous la marque Leontopod stems GX® contenant 20% de cellules, 0.3% de gomme xanthane et pour le reste de la glycérine

### Exemple 1 : Action anti-radicalaire (in vitro) :

La peroxydation lipidique est un cas typique de réaction en chaîne induite par voie radicalaire. L'oxydation des lipides membranaires aboutit à la formation de lipoperoxydes qui se décomposent en différents produits de fragmentation dont certains sont très toxiques et agressifs pour la peau.

L'un des produits de fragmentation le plus important et le plus agressif est un aldéhyde, le malone dialdéhyde (MDA) qui manifeste une toxicité redoutable en pontant transversalement les proteines, les lipides intracellulaires et l'ADN.

Il apparaît donc que les radicaux libres et la cascade de réaction en chaîne qu'ils provoquent au sein de l'organisme, jouent un rôle essentiel dans le processus de vieillissement cutané.

A cet effet chacun des deux actifs a été soumis au traitement d'initiation de la peroxydation lipidique suivante : La peroxydation d'une émulsion d'acide linoléique a été induite par des radicaux hydroxyle °OH.

Pour mesurer la peroxydation des lipides, la Demanderesse a réalisé un dosage in vitro des complexes entre les produits d'oxydation lipidiques et l'acide thiobarbituriques selon Morlière P et al. Biochim Biophys, Acta, 1991; 1084, p 261-268.

Ces complexes sont appelés TBARS (Thiobarbituric Acid Reactive Substances).

Afin de reproduire les effets d'un stress oxydatif, la Demanderesse a traité pendant 1 heure une lignée de fibroblastes L 929, par un complexe composé de peroxyde d'hydrogène et de fer, reconstituant ainsi la réaction de Fenton source de RLO et plus particulièrement de radical hydroxyle . Les résultats sont mentionnés dans le tableau 1.

**Tableau 1 : Effet du rétinaldéhyde, de l'extrait de leontopodium alpinum et de la composition selon l'invention (rétinaldéhyde + extrait de leontopodium alpinum) sur la protection contre le stress oxydatif et notamment la peroxydation lipidique (exprimé en %)**

| Produit testé | Gain de protection observé contre la peroxydation lipidique (en %) |
|---|---|
| Rétinaldéhyde seul (0,05% en poids) | 32% |
| Extrait de *Leontopodium alpinum* (0,5% en poids) | 17% |
| Rétinaldéhyde (0,05 à 0,2 % en poids) + Extrait de *leontopodium alpinum* (0,2 à 0, 5 % en poids) | 52 à 195 % |

Ces résultats montrent donc un effet synergique du rétinaldéhyde et de l'extrait de *leontopodium alpinum* pour la protection du stress oxydatif.

Le meilleur résultat est obtenu avec la dose de 0,2 % de rétinaldéhyde avec la dose de 0,5 % d'extrait glycériné (195 % de protection contre la péroxydation lipidique)

A la dose de 0,05 % de rétinaldéhyde avec 0,5 % d'extrait glycériné de *Leontopodium alpinum,* le gain de protection est encore de 98 % .

A la dose de 0,1 % de Rétinaldéhyde avec 0,5 % d'extrait glycériné de Leontopodium alpinum le gain de protection lipidique est de 132 %. Le rétinaldéhyde seul à la dose de 0,1 % donne un gain de protection de 52 % .

Ces résultats démontrent l'effet synergique de l'association:
- Rétinaldéhyde 0,05 % et 0,5 % d'extrait glycériné de Leontopodium alpinum soit 98 %.
alors que l'effet additif est de seulement : 32 % + 17 % soit 49 %.

Avec la dose de 0,1 % de rétinaldéhyde en présence de 0,5% d'extrait glycériné de *Leontopodium alpinum* le gain est de 132 %, alors que l'effet additif est de 52 % + 17 % soit 69 %.

### Exemple 2 : Régénération du collagène (culture cellulaire in vitro)

L'étude est réalisée par mesure de l'incorporation de proline radioactive dans des cultures de fibroblastes dermiques humains.

Les cultures de fibroblastes sont effectuées selon les méthodes classiques de culture cellulaire , à savoir en milieu MEM/M199 vendu par la société GIBCO, en présence de bicarbonate de sodium (1,87 mg/ml) de 1 glutamine (2mM), de pénicilline (50UI/ml) et de 10 % de sérum de veau fœtal (Gibco).

Le test est effectué sur des cultures de cellules à 80 % de confluence en plaque de 24 trous. Les produits sont mis en contact avec les cellules pendant 48 heures. Le marquage à la proline tritiée vendue par Amersham est effectué pendant 24 heures. Les résultats sont mentionnés dans le tableau 2.

Dans ces conditions expérimentales, nous avons observé, une forte potentialisation de la régénération du collagène avec l'association rétinaldéhyde et l'extrait de *leontopodium alpinum.*

**Tableau 2 : Effet du rétinaldéhyde, de l'extrait de Leontopodium alpinum et de la composition selon l'invention (rétinaldéhyde + extrait de Leontopodium alpinum) sur la régénération du collagène (exprimé en %)**

| | Effet sur la régénération du collagène (en %) |
|---|---|
| Rétinaldéhyde seul (0,05% en poids) | 27% |
| Extrait de *leontopodium alpinum* (0,5% en poids) | 11% |
| Rétinaldéhyde (0,05 à 0,1 % en poids) + Extrait de *leontopodium alpinum* (0,1 à 0, 5 % en poids) | 58 à 168 % |

Ces résultats montrent donc un effet synergique du rétinaldehyde et de l'extrait de *leontopodium alpinum* sur la régénération du collagène.

Le meilleur résultat est obtenu avec 0,1 % de rétinaldéhyde et 0,5 % d'extrait glycériné de *Leontopodium alpinum* (168 % de régénération de collagène)

A la dose de 0,05 % de rétinaldéhyde et 0,5 % d'extrait glycériné de Leontopodiumalpinum le gain de régénération est de 102%.

A la dose de 0,1 % le rétinaldéhyde donne 55 % de régénération. Ces résultats confirment l'effet synergique de l'association :
- Rétinaldéhyde 0,05 % avec 0,5 % d'extrait glycériné de Leontopodium alpinum gain de 102 %, alors que l'effet additif est de : 27 %= 11 % soit 38 % .
- Rétinaldéhyde 0,1 % avec 0, 5 % d'extrait glycériné de Leontopodium alpinum gain de 168 %, alors que l'effet additif est de : 55 % + 11 % soit 66 % .

### Exemple 3 : essai clinique

Les 3 groupes sont testés avec les même ingrédients et % de la formulation décrite dans l'exemple 3 .Seule la composition des actifs est différente :
1er groupe : un seul actif le rétinaldéhyde à la dose de 0,05 % en poids
2ème groupe : un seul actif l'extrait glycériné de Leontopodium alpinum à la dose de 0,5 % en poids .

### Exemple 3 : Etudes cliniques : action anti-rides de la composition

La formulation ci-dessous a été testée en clinique sous contrôle dermatologique en utilisant une méthode d'appréciation de l'activité anti-rides.

**Formulation sous forme de crème émulsion H/E :**

| Composants | Quantité en poids |
|---|---|
| Rétinaldéhyde trans-cis | 0,05g |
| Extrait de racine de *Leontopodium alpinum* | 500 mg |
| Triglycérides/capric/caprylic 30- 70 | 8 à 10 g |
| Monostearate glycerol PEG 100 | 4 à 6 g |
| Paraffine liquide | 3 à 5 g |
| Propylene glycol | 3 à 5 g |
| Squalane | 1 à 3 g |
| Sepigel 305 | 1 à 2 g |
| Synthalen K | 0,2 à 0,5g |
| Triethanolamine | 0,2 à 0,5g |
| Colorant | 0,2 à 2 g |
| méthylisothiazolinone | 0,01 g |
| Eau purifiée QSP | 100 g |

La méthode utilisée est la technique des empreintes cutanées associée à l'analyse macro-photographique.

L'analyse du microrelief cutané a été effectuée par réalisation d'empreintes au niveau du visage avec un polymère siliconé (SILFLO) sur 3 groupes de femmes :
Le 1er groupe (20 femmes) est traité par 2 g de crème contenant comme actif uniquement le rétinaldéhyde , chaque jour pendant 90 jours.
Le 2ème groupe (20 femmes) est traité par 2 g de crème contenant comme actif uniquement l'extrait de racine de Leontopodium alpinum.
Le 3ème groupe (25 femmes) est traité par 2 g de crème contenant l'association des 2 actifs.

Les mesures sont effectuées chaque semaine avec le Skin Image Analyser selon le protocole suivant : la surface de la réplique siliconée a été éclairée en lumière rasante à 35° pour générer des ombres portées qui ont ensuite été observées à l'aide d'une caméra CCD, relié à un système informatique.

L'image numérique obtenue a été analysée pour obtenir les différents paramètres caractérisant le relief de la surface cutanée du visage. Les résultats de cette étude sont mentionnés dans le tableau 3.

Les résultats de cette étude effectuée sur 65 femmes de 27 à 85 ans pendant une durée de trois mois, sont mentionnés en % de diminution des rides dans le tableau 3.

**Tableau 3 : Effet du rétinaldéhyde, de l'extrait de leontopodium alpinum et de la composition selon l'invention (rétinaldéhyde + extrait de leontopodium alpinum) sur la diminution de l'apparition des rides (exprimé en %)**

| Produit testé | Rétinaldéhyde seul | | | Extrait de racine de *Leontopodium alpinum* seul | | | Rétinaldéhyde + Extrait de racine de *Leontopodium alpinum* | | |
|---|---|---|---|---|---|---|---|---|---|
| Temps (en mois) | 1 | 2 | 3 | 1 | 2 | 3 | 1 | 2 | 3 |
| Rides légères | 9% | 12% | 19% | 6% | 9% | 12% | **34%** | **58%** | **79%** |
| Rides moyennes | 6% | 9% | 14% | 4% | 6% | 10% | **18%** | **32%** | **61%** |
| Rides profondes | 3% | 4% | 5% | 2% | 3% | 3% | **12%** | **19%** | **32%** |

L'utilisation de la composition selon l'invention permet de réduire l'apparition des rides de l'ordre de 79% pour les rides légères, de l'ordre de 61% pour les rides moyennes et de l'ordre de 32% pour les rides profondes.

Ces résultats confirment donc la très forte potentialisation de l'action antirides de l'association rétinaldéhyde avec l'extrait de *Leontopodium alpinum.*

### Exemple 4 : Préparation d'une crème H/E

Cet exemple illustre la préparation d'une crème H/E comprenant un rétinaldéhyde tout-trans et un extrait de Leontopodium alpinum.

| Composants | Quantité en poids |
|---|---|
| Rétinaldéhyde tout-trans | 0,1g |
| Extrait de *Leontopodium alpinum* | 1 g |
| Triglycérides /capric/caprylic | 13 à 18 g |
| Monostearate glycerol | 7 à 10 g |
| Paraffine liquide | 4 à 6 g |
| Propylene glycol | 2 à 3 g |
| Squalane | 3 à 5 g |
| Cire E poudre | 1 à 2 g |
| Carbopol 934 | 0,3 à 0,5g |
| Parahydroxybenzoate de méthyle | 0,2 à 0,4g |
| Parahydroxybenzoate d' éthyle | 0,2 à 0,4 g |
| Colorant | 0,2 à 1 g |
| Parfum hypoallergènique | 0,2 à 1 g |
| Eau purifiée QSP | 100 g |

### Exemple 5: Préparation d'un gel pour le traitement de l'acné et des dermites séborrheiques.

Cet exemple illustre la préparation d'un gel comprenant un rétinaldéhyde trans-cis, un extrait de fleur de *Leontopodium alpinum* et de l'acide azélaique, pour le traitement de l'acné et des dermites séborrheiques.

| Composants | Quantité en poids |
|---|---|
| Rétinaldéhyde trans-cis | 0,1 g |
| Extrait de fleur de *Leontopodium alpinum* | 0,5 g |
| Acide azélaique | 0,1 à 2 g |
| Macrogol 600P | 4 à 5 g |
| Paraffine liquide | 4 à 5 g |
| Alcool 95° | 2 à 4 g |
| Huile de ricin hydrogénée éthoxylée | 1 à 3 g |
| Carbopol 940 | 1 à 2 g |
| Alpha tocophérol acétate | 0,5 à 1 g |
| Parahydroxybenzoate de méthyle | 0,3 à 0,5 g |
| Colorant | 0,2 à 1 g |
| Conservateur ECOCERT | 0,2 à 1 g |
| Parfum hypoallergénique | 0,2 à 1 g |
| Eau purifiée QSP | 100 g |

### Exemple 6: Préparation d'une crème pour le traitement du prurit et des dermites atopiques

Cet exemple illustre la préparation d'une crème comprenant un rétinaldéhyde trans-cis, un extrait de fleur et de feuille de *Leontopodium alpinum* et du crotamiton, pour le traitement du prurit et des dermites atopiques.

| Composants | Quantité en poids |
|---|---|
| Rétinaldéhyde trans-cis | 0,2g |
| Extrait de fleur et de feuille de *Leontopodium alpinum* | 1 g |
| Crotamiton | 0,1 à 2 g |
| Huile de carthame hybride | 8 à 12 g |
| Neobee M5 | 8 à 12 g |
| Monostearate de glycérol POEG | 7 à 10 g |
| Paraffine liquide | 4 à 6 g |
| Propylène glycol | 2 à 4 g |
| Colorant | 0,2 à 1 g |
| Conservateur ECOCERT | 1 à 2 g |
| Parfum hypoallergènique | 0,2 à 1 g |
| Eau purifiée QSP | 100 g |

### Exemple 7: Préparation d'un gel pour le traitement des zones hyperpigmentées et les taches brunes

Cet exemple illustre la préparation d'un gel comprenant un rétinaldéhyde tout-trans, un extrait de fleur, feuille et tige de *Leontopodium alpinum* et de l'arbutine, pour le traitement des zones hyperpigmentées et les taches brunes.

| Composants | Quantité en poids |
|---|---|
| Rétinaldéhyde tout-trans | 0,05 à 2 g |
| Extrait de fleur, feuille et tige de *Leontopodium alpinum* | 0,1 à 2 g |
| Arbutine | 0,1 à 2 g |
| Polyoxyethylene glycol 600 | 2 à 8 g |
| Paraffine liquide | 2 à 8 g |
| Alcool 95° | 1 à 2 g |
| Huile de ricin hydrogénée éthoxylée 40 moles | 1 à 2 g |
| Colorant | 0,2 à 1 g |
| Conservateur ECOCERT | 0,2 à 1 g |
| Parfum hypoallergènique | 0,2 à 1 g |
| Eau purifiée QSP | 100 g |

### Exemple 8: Préparation d'une crème hydratante.

Cet exemple illustre la préparation d'une crème hydratante comprenant un rétinaldéhyde trans-cis, une culture de cellule de méristème de *Leontopodium alpinum* et de l'hyaluronate de sodium.

| Composants | Quantité en poids |
|---|---|
| Rétinaldéhyde trans-cis | 0,01 à 0,05 g |
| Culture de cellule de méristème de *leontopodium alpinum* | 0,1 à 0,5 g |
| Hyaluronate de sodium | 0,1 à 2 g |
| Huile de Vaseline | 30 à 40 g |
| Cire d'abeille | 15 à 20 g |
| Lanoline | 8 à 12 g |
| Monostearate de sorbitan | 1 à 2 g |
| Monostearate de sorbitan ethoxylé | 2 à 3 g |
| Colorant | 0,2 à 1 g |
| Conservateurs | 0,1 à 0,5 g |
| Eau purifiée QSP | 100 g |

## Revendications

1. Une composition comprenant au moins un rétinaldéhyde et/ou un de ses stéréoisomères et de 0,01 à 2% en poids d'une préparation de cellules d'une culture de cellules de méristème de *leontopodium alpinum* en présence de glycérine et de gomme xanthane, disponible sous la marque Leontopod stems GX® contenant 20% de cellules, 0.3% de gomme xanthane et pour le reste de la glycérine, par rapport au poids total de la composition.

2. La composition selon la revendication 1, dans laquelle le rétinaldéhyde est sélectionné parmi le groupe comprenant : le rétinaldéhyde trans-cis, le rétinaldéhyde tout-trans ou un mélange de ses deux stéréoisomères.

3. La composition selon l'une des revendications 1 à 2, comprenant de 0,01 à 2% en poids, de préférence de 0,05 à 0,1% en poids de rétinaldéhyde par rapport au poids total de la composition.

4. La composition selon l'une des revendications 1 à 3, comprenant de 0,05 à 2% en poids d'une préparation de cellules d'une culture de cellules de méristème de *leontopodium alpinum* en présence de glycérine et de gomme xanthane, disponible sous la marque Leontopod stems GX® contenant 20% de cellules, 0.3% de gomme xanthane et pour le reste de la glycérine, par rapport au poids total de la composition.

5. La composition selon l'une des revendications 1 à 4, comprenant en outre au moins un principe actif supplémentaire sélectionné parmi le groupe consistant en : le crotamiton, l'acide azélaïque, l'arbutine et le hyaluronate de sodium.

6. La composition selon la revendication 5, dans laquelle le hyaluronate de sodium est un hyaluronate de sodium de poids moléculaire supérieur à 750000 daltons.

7. La composition selon la revendication 5, dans laquelle le principe actif supplémentaire est présent entre 0,1 et 2% en poids, de préférence de 0,5 à 1% en poids par rapport au poids total de la composition.

8. Utilisation de la composition selon l'une des revendications 1 à 7 pour réduire les troubles de la sénescence cutanée.

9. Composition selon l'une des revendications 1 à 7 pour son utilisation dans le traitement de l'acné.

10. Composition selon l'une des revendications 1 à 7, se présentant sous forme aqueuse comprenant une phase aqueuse, ladite phase aqueuse comprenant un ou plusieurs solvants organiques.

11. Composition selon la revendication 10, dans laquelle les solvants organiques sont choisis parmi les alcools, tels que l'éthanol, l'isopropanol, le tert-butanol, le n-butanol, les polyols, tels que la glycérine, le propylène glycol, le butylène glycol, l'isoprène glycol, le polyéthylène glycol, le macrogol, les éthers de polyol.

## Patentansprüche

1. Zusammensetzung, wenigstens umfassend ein Retinaldehyd bzw. eines seiner Stereoisomere und 0,01 bis 2 Gew.-% einer Zell-Zusammensetzung einer Meristem-Zellkultur von *leontopodium alpinum* (Alpen-Edelweiß) in Anwesenheit von Glycerin und Xanthangummi, erhältlich unter der Marke Leontopod Stems GX® mit 20 % Zellen, 0,3 % Xanthangummi und Rest als Glycerin im Verhältnis zum Gesamtgewicht der Zusammensetzung.

2. Zusammensetzung nach Anspruch 1, wobei das Retinaldehyd aus folgender Gruppe ausgewählt ist: trans-cis-Retinaldehyd, all-trans-Retinaldehyd oder einer Mischung seiner beiden Stereoisomere.

3. Zusammensetzung nach einem der Ansprüche 1 - 2 mit 0,01 bis 2 Gew.-%, bevorzugt 0,05 bis 0,1 Gew.-% Retinaldehyd im Verhältnis zum Gesamtgewicht der Zusammensetzung.

4. Zusammensetzung nach einem der Ansprüche 1 bis 3 mit 0,05 bis 2 Gew.-% einer Zell-Zusammensetzung einer Meristem-Zellkultur von *leontopodium alpinum* in Anwesenheit von Glycerin und Xanthangummi, erhältlich unter der Marke Leontopod Stems GX® mit 20 % Zellen, 0,3 % Xanthangummi und Rest als Glycerin im Verhältnis zum Gesamtgewicht der Zusammensetzung.

5. Zusammensetzung nach einem der Ansprüche 1 bis 4, ferner umfassend einen zusätzlichen Wirkstoff, ausgewählt aus der Gruppe bestehend aus: Crotamiton, Azelainsäure, Arbutin und Natriumhyaluronat.

6. Zusammensetzung nach Anspruch 5, wobei das Natriumhyaluronat ein Molekulargewicht von über 750 000 Dalton hat.

7. Zusammensetzung nach Anspruch 5, wobei der zusätzliche Wirkstoff mit 0,1 bis 2 Gew.-%, bevorzugt mit 0,5 bis 1 Gew.-%, im Verhältnis zum Gesamtgewicht der Zusammensetzung vorliegt.

8. Verwendung der Zusammensetzung nach einem der Ansprüche 1 bis 7 zur Minderung der Probleme der Hautalterung.

9. Zusammensetzung nach einem der Ansprüche 1 bis 7 für ihre Verwendung zur Aknebehandlung.

10. Zusammensetzung nach einem der Ansprüche 1 bis 7 in wässriger Form, die eine Wasserphase umfasst, wobei die Wasserphase ein oder mehrere organische Löschungsmittel enthält.

11. Zusammensetzung nach Anspruch 10, wobei die organischen Lösungsmittel ausgewählt sind aus Alkohol, z. B. Ethanol, Isopropanol, tert-Butanol, n-Butanol, Polyole, z. B. Glycerin, Propylenglycol, 1,3-Butandiol, Isoprenglycol, Polyethylenglycol, Macrogol, Polyether.

## Claims

1. A composition comprising at least retinaldehyde and/or a stereoisomer thereof and from 0.01 to 2 wt % of a cell preparation from a *Leontopodium alpinum* meristem cell culture together with glycerine, xanthan gum, available under the trade mark Leontopod stems GX® comprising 20% of cells, 0.3% of xanthan gum and the remainder being glycerine, with respect to the total weight of the composition.

2. The composition according to the claim 1, wherein retinaldehyde is selected from the group consisting of : trans-cis retinaldehyde, all-trans retinaldehyde or a mixture of these two stereoisomers.

3. The composition according to one of the claims 1 to 2, comprising from 0.01 to 2% by weight, preferably from 0.05 to 0.1% by weight of retinaldehyde with respect to the total weight of the composition.

4. The composition according to one of claims 1 to 3, comprising from 0.05 to 2% by weight of a cell preparation from a *Leontopodium alpinum* meristem cell culture together with glycerine, xanthan gum, available under the trade mark Leontopod stems GX® comprising 20% of cells, 0.3% of xanthan gum and the remainder being glycerine, with respect to the total weight of the composition.

5. The composition according to one of claims 1 to 4, further comprising at least one additional active ingredient selected from the group consisting of: crotamiton, azelaic acid, arbutin and sodium hyaluronate.

6. The composition according to claim 5, in which the sodium hyaluronate is a sodium hyaluronate of a molecular weight above 75000 Dalton.

7. The composition according to claim 5, in which the additional active ingredient is present between 0.1 and 2% by weight, preferably from 0.5 to 1% by weight with respect to the total weight of the composition.

8. Use of the composition according to one of the claims 1 to 7 for reducing skin senescence disorders.

9. Composition according to one of claims 1 to 7 for its use in treating acne.

10. Composition according to one of claims 1 to 7, being present in aqueous form comprising an aqueous phase, said aqueous phase comprising one or several organic solvents.

11. Composition according to the claim 10, wherein the organic solvents are selected from alcohols, such as ethanol, isopropanol, tert-butanol, n-butanol, polyols, such as glycerin, propylene glycol, butylene glycol, isoprene glycol, polyethylene glycol, macrogol and polyol ethers.
